# EUROPEAN PATENT SPECIFICATION

(11) **EP 4 188 278 B1**
(45) Date of publication and mention of the grant of the patent: **28.01.2026**
(21) Application number: 21758520.7
(22) Date of filing: 29.07.2021
(51) Int. Cl.: A61F 2/08, A61B 17/064

(54) **MEDICAL IMPLANT DELIVERY SYSTEM**
SYSTEM ZUR EINFÜHRUNG EINES MEDIZINISCHEN IMPLANTATS
SYSTÈME DE POSE D'IMPLANT MÉDICAL

(30) Priority: 03.08.2020 US 202063060466 P
(43) Date of publication of application: 07.06.2023
(73) Proprietor: Smith & Nephew, Inc., Memphis, TN 38116 (US); Smith & Nephew Orthopaedics AG, 6300 Zug (CH); Smith & Nephew Asia Pacific Pte. Limited, Singapore 138565 (SG)
(72) Inventor: GRABINSKY, Jessica M., Andover, Massachusetts 01810 (US); YEUNG, David A., Andover, Massachusetts 01810 (US); TORRES, Francheska, Charlton, Massachusetts 01507 (US)
(74) Representative: Vossius & Partner Patentanwälte Rechtsanwälte mbB
(86) International application number: PCT/US2021/043668
(87) International publication number: WO 2022/031511

(56) References cited:
- WO-A1-2017/117415
- WO-A2-2011/128903
- US-A1- 2015 320 543

## Description

### TECHNICAL FIELD

The present disclosure pertains generally, but not by way of limitation, to orthopedic implants and methods of treatment. More particularly, the present disclosure relates to a tendon repair implant, such as one that is engineered for arthroscopic placement over or in the area of a full or partial thickness tear of the supraspinatus tendon of the shoulder or other tendon, and an associated delivery device.

### BACKGROUND

With its complexity, range of motion and extensive use, a common soft tissue injury is damage to the rotator cuff or rotator cuff tendons. Damage to the rotator cuff is a potentially serious medical condition that may occur during hyperextension, from an acute traumatic tear or from overuse of the joint. Current procedures for treatment of a torn tendon include affixing a biocompatible implant over the torn tendon. There is an ongoing need to deliver and adequately position medical implants during an arthroscopic procedure in order to treat injuries to the rotator cuff, rotator cuff tendons, or other soft tissue or tendon injuries throughout a body.

US 2015/0320543 A1 discloses an implant assembly for introducing and positioning implants within patients, the assembly comprising an implant device, an implant and a sheath. The implant device includes an upper beam, a lower beam and an implant positioning component. The implant is disposed between the upper beam and the lower beam and includes a first face engaged with the upper beam and a second face engaged with the lower beam. The implant is at least partially disposed around the implant positioning component.

### BRIEF SUMMARY

This disclosure provides design, material, manufacturing method, and use alternatives for medical devices. An example implant delivery system includes a delivery shaft including a proximal portion and a distal portion, a frame coupled to the distal portion of the delivery shaft, the frame including a body portion and at least first and second arms, the first and second arms each having first ends attached to the body portion and second, free ends opposite the first ends, the first arm having a first tab disposed adjacent its free end, the second arm having a second tab disposed adjacent its free end, and the body portion having a third tab, wherein the first tab extends toward the free end of the first arm and the second tab extends toward the body portion, and wherein the first, second, and third tabs are all configured to be releasably attached to an implant.

Alternatively or additionally to any of the embodiments above, the first and second tabs include a sharp tip configured to extend at least partially through the implant.

Alternatively or additionally to any of the embodiments above, the body portion and first and second arms define a Y shape.

Alternatively or additionally to any of the embodiments above, the first and second arms each extend at an angle from the body portion such that when the third tab engages an edge of an implant, the free ends of the first and second arms are disposed adjacent opposing edges of the implant.

Alternatively or additionally to any of the embodiments above, the third tab extends toward the free ends of the first and second arms.

Alternatively or additionally to any of the embodiments above, the third tab is blunt and configured to engage an edge of the implant.

Alternatively or additionally to any of the embodiments above, the third tab includes a sharp tip configured to extend at least partially through the implant.

Alternatively or additionally to any of the embodiments above, the frame has a concave shape between the body portion and the free ends of the first and second arms.

Alternatively or additionally to any of the embodiments above, the frame has an upper surface and a lower surface, and the first and second tabs extend downward from the lower surface.

Alternatively or additionally to any of the embodiments above, the first and second arms are fixed to the body portion and have a fixed length.

Alternatively or additionally to any of the embodiments above, an entirety of the frame, including the body portion and first and second arms, is formed from a single, monolithic piece.

Alternatively or additionally to any of the embodiments above, the implant delivery system further comprises a tack member connected to the body portion.

Alternatively or additionally to any of the embodiments above, the frame is removably coupled to the delivery shaft, and configured to be detached from the delivery shaft in vivo.

Alternatively or additionally to any of the embodiments above, the implant delivery system further comprises a tether extending through a lumen of the delivery shaft, the tether fixedly attached to the frame such that the tether remains attached to the frame when the delivery shaft is detached from the frame.

Another implant delivery system includes a delivery shaft including a proximal portion and a distal portion, a frame coupled to the distal portion of the delivery shaft, the frame including a body portion and at least first and second arms extending from the body portion, the frame including a plurality of tabs configured to releasably engage an implant, the first and second arms each having free ends, the free end of the first arm defining a first tab, the free end of the second arm defining a second tab, and the body portion having a third tab, wherein the first, second, and third tabs are configured to engage at least one edge of the implant.

Alternatively or additionally to any of the embodiments above, the free ends of the first and second arms are bent such that when the first and second arms are disposed on an upper surface of a planar implant, the free ends of the first and second arms wrap around side edges of the implant forming first and second tabs that extend along a lower surface of the implant.

Alternatively or additionally to any of the embodiments above, the body portion and first and second arms define a Y shape.

Alternatively or additionally to any of the embodiments above, the first and second arms each extend at an angle from the body portion such that when the third tab engages the at least one edge of the implant, the free ends of the first and second arms are disposed adjacent the at least one edge at opposing edges of the implant.

Alternatively or additionally to any of the embodiments above, when the implant is a polygon and the third tab engages a first edge of the implant, the first and second tabs of the first and second arms are configured to engage second and third edges of the implant adjacent the first edge of the implant.

Alternatively or additionally to any of the embodiments above, when the implant is circular and has an outer edge, and the third tab engages a first location on the outer edge, the first and second tabs of the first and second arms are configured to engage second and third locations on the outer edge of the implant spaced apart from the first location.

An example implant assembly includes a one-piece planar implant, and a delivery system including an outer shaft including a proximal end, a distal end, and a lumen extending therebetween, an inner shaft slidably disposed within the lumen of the outer shaft, and a frame fixedly attached to a distal end of the inner shaft and configured to be releasably attached to the implant, the frame including a body portion and at least first and second arms extending from the body portion, each of the first and second arms having a free end configured to penetrate the implant when inserted through the implant in a first direction, and to resist withdrawal from the implant when pulled in a second direction opposite the first direction, wherein the first and second arms are woven into and out of the implant.

Alternatively or additionally to any of the embodiments above, the free ends of the first and second arms each include a sharp distal tip and a flared region proximal of the sharp distal tip.

Alternatively or additionally to any of the embodiments above, the frame, including the body portion and first and second arms, is formed from as a single, monolithic piece.

Alternatively or additionally to any of the embodiments above, the frame defines a single concave arc between the body portion and the free ends of the first and second arms.

Alternatively or additionally to any of the embodiments above, the first and second arms are fixed to the body portion and have a fixed length.

Alternatively or additionally to any of the embodiments above, the body portion and first and second arms define a Y shape.

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The disclosure may be more completely understood in consideration of the following detailed description in connection with the accompanying drawings, in which:
Figure 1 illustrates a cross-section of an anterior view of a shoulder of a patient;
Figure 2 illustrates a shoulder including a head of the humerus mating with the glenoid fossa of the scapula at a glenohumeral joint and an implant delivery system positioned over a tendon;
Figure 3 illustrates an example implant delivery device attached to an implant;
Figure 4A is a cross-sectional view taken along line A-A in Figure 3;
Figure 4B is a cross-sectional view taken along line B-B in Figure 3;
Figure 5A illustrates a side view of the example implant delivery device of Figure 3 with the sheath in cross-section;
Figure 5B illustrates an end view along line 5B-5B in Figure 5A;
Figures 6-10 illustrate an exemplary method of installing an implant with an example implant delivery device at a target site;
Figure 11 illustrates another example implant delivery device attached to an implant;
Figure 12 illustrates another example implant delivery device attached to an implant;
Figure 13A is a cross-sectional view taken along line 13-13 in Figure 12 without the implant;
Figure 13B is a cross-sectional view taken along line 13-13 in Figure 12;
Figure 14 illustrates another example implant delivery device attached to an implant; and
Figure 15 illustrates another example implant delivery device attached to an implant.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the scope of the claimed invention, as defined the claims.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the disclosure.

Rotator cuff surgeries are most frequently performed arthroscopically through various portals outside the shoulder. In order to successfully complete the repairs, the surgeons require appropriately sized medical devices that fit through the skin portals created. An accepted treatment for rotator cuff tears includes reattaching the torn tendon to the humeral head using sutures. Additionally, in treating rotator cuff tears, an accepted practice may also include the placement of a scaffold or planar implant over the repaired tendon to mechanically reinforce the repaired tendon. In order to fix the implant to the native tendon, a delivery device including a sheath may be used to shield the implant from the soft tissue during insertion into the joint. Once inside the joint space, the implant may be deployed from the sheath. Sufficient control of the implant is needed for manipulation inside the joint space. Additionally, a secure yet releasable connection between the delivery device and the implant is needed to successfully complete the procedure.

It is noted that although the disclosure below is in reference to treatment of a tendon of the shoulder joint, it is contemplated and within the understanding of this disclosure that the implants, delivery devices, and uses thereof, are also applicable to other body joints, such as the hip, knee, and elbow, as well as other regions of the body.

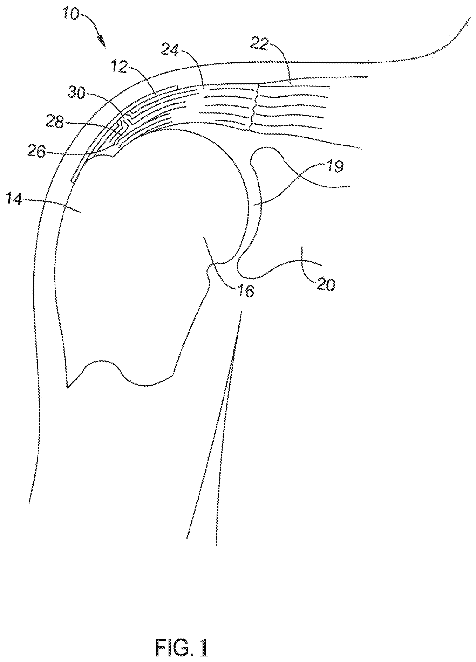

Figure 1 shows a cross-sectional view of a shoulder 10 including an example implant 12. Shoulder 10 further shows a head 14 of humerus 16 mating with a glenoid fossa 19 of scapula 20. The glenoid fossa 19 comprises a shallow depression in scapula 20. A supraspinatus tendon 22 is also shown. These muscles (along with others) control the movement of humerus 16 relative to scapula 20. A distal tendon 24 of supraspinatus tendon 22 meets humerus 16 at an insertion point 26.

In Figure 1, tendon 24 includes a damaged portion 28 located near insertion point 26. Damaged portion 28 includes a tear 30 extending partially through tendon 24. Tear 30 may be referred to as a partial thickness tear. However, in other instances the tear 30 may be a full thickness tear. The depicted partial thickness tear 30 is on the bursal side of the tendon, however, the tear may also be on the opposite or articular side of the tendon 24 and/or may include internal tears to the tendon 24 not visible on either surface.

Figure 1 further illustrates that the tendon repair implant 12 has been placed over the partial thickness tear 30. In this example, the tendon repair implant 12 is placed on the bursal side of the tendon regardless of whether the tear is on the bursal side, articular side or within the tendon. Further, the tendon repair implant 12 may overlay multiple tears.

In some instances, delivery of an implant 12 (e.g., a sheet-like, one-piece planar implant) to a target site of a patient may require a physician to create an incision in the patient sufficient to access the target implant site. After creating this "access site," the physician may insert an implant delivery system through the access site and position the distal end of the implant delivery system adjacent the target implant site. The physician may then manipulate the implant delivery system to deploy an implant out of a delivery sheath or otherwise deploy the implant adjacent the target implant site.

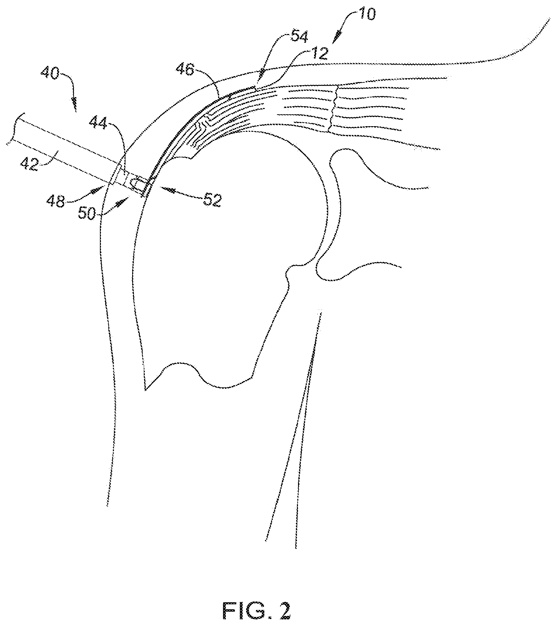

For example, Figure 2 provides a perspective view of an implant delivery system 40 extending through the shoulder 10 of a patient. Figure 2 shows implant delivery system 40 deployed adjacent a target site (e.g., a tear in the supraspinatus tendon). The implant delivery system 40 may be substantially similar to the implant delivery system disclosed in U.S. Patent No. 10,314,689. In at least some embodiments, implant delivery system 40 comprises a delivery sheath 42 (e.g., a cannula) including a proximal portion (not shown), a distal end 48 and a lumen extending within at least a portion of delivery sheath 42. Further, implant delivery system 40 may include a delivery shaft 44 extending within the lumen of delivery sheath 42 and longitudinally movable relative thereto.

Delivery shaft 44 may include a proximal portion (not shown) extending out of the proximal portion of delivery sheath 42 and/or otherwise manipulatable relative to delivery sheath 42 by a user. Additionally, in some examples the proximal portion of delivery shaft 44 and/or or delivery sheath 42 may be coupled to a handle member (not shown). The handle member may be utilized to manipulate delivery shaft 44. For example, the handle member may be utilized to impart a rotational and/or longitudinal force to delivery shaft 44.

In addition, delivery shaft 44 may include a distal portion 50 extending out of the distal end 48 of delivery sheath 42. Further, delivery shaft 44 may include a lumen extending therein. The lumen of delivery shaft 44 may extend along a portion or the entire length of delivery shaft 44 (e.g., from distal portion 50 to the proximal portion of delivery shaft 44).

Delivery system 40 may further include a frame 46 coupled to the distal portion 50 of the delivery shaft 44. In some examples, the frame 46 may be fixedly mounted to the delivery shaft 44. As shown in Figure 2, frame 46 may be detachably coupled to an implant 12 (e.g., a sheet-like, planar implant). For purposes of the discussion herein, the combined structure including frame 46 and implant 12 may be defined as having a proximal portion 52 and a distal portion 54 as illustrated in Figure 2.

When initially positioning the frame 46 and implant 12 adjacent a target site, a clinician may orient the frame 46 and implant 12 (for example, via a handle member attached to a proximal portion of the delivery shaft 44) such that the proximal portion 52 may be adjacent (e.g., overlaid) on a portion of the humerus (e.g., on the bone), while the distal portion 54 of the frame 46 and implant 12 may overlay the tendon 24.

As described above, delivery of implant delivery system 40 may include the insertion of delivery sheath 42 through an access site (e.g.. incision) and advancement to a target site. After positioning the distal end 48 of delivery sheath 42 proximate the target site, a clinician may deploy the frame 46 in combination with the implant 12 out of the lumen located within and along the distal end 48 of the delivery sheath 42, such as by retracting delivery sheath 42 relative to delivery shaft 44 and frame 46, and positioning implant 12 and frame 46 over the target site.

Prior to deployment, the frame 46 and implant 12 combination may be contained (e.g., housed) within the lumen of delivery sheath 42 for subsequent deployment distally out distal opening of delivery sheath 42. As will be described in greater detail below, the combination of frame 46 and implant 12 may wrap and/or fold upon itself such that it may be positioned within the lumen of the delivery sheath 42. Alternatively, frame 46 and implant 12 may wrap and/or fold around implant delivery shaft 44 while disposed within delivery sheath 42. For example, frame 46 may be formed of a flexible material, such as a flexible polymer or metal material, capable of undergoing elastic deformation to collapse frame 46 for placement within the lumen of the delivery sheath 42.

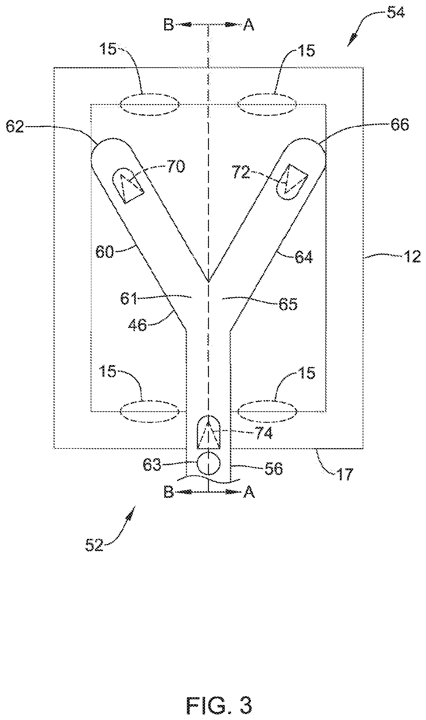

Figure 3 shows an example frame 46 coupled to example implant 12. As stated above with reference to Figure 2, frame 46 and implant 12 may have a proximal portion 52 which, for purposes of discussion herein, may be adjacent delivery shaft 44 and be configured to be positioned adjacent humerus 16. Further, frame 46 and implant 12 may have a distal portion 54 which, for purposes of discussion herein, may extend away from delivery shaft 44 and be configured to be positioned adjacent tendon 24.

Figure 3 further shows fastening regions 15 located at various positions within implant 12. As shown in Figure 3, the fastening regions 15 are positioned at locations which are free from the structure of frame 46. In other words, the shape of frame 46 may be designed to specifically permit fastening implant 12, such as with staples, to the anatomy at fastening regions 15 while frame overlies and is secured to implant 12.

As shown in Figure 3, frame 46 may include a body portion 56, first arm 60 and second arm 64. The body portion 56 may be positionable along a central portion of the implant 12 with first and second arms 60, 64 extending laterally from the body portion 56 in opposite directions toward opposite side edges of the implant 12. The first arm 60 may have a first end 61, or base, attached to the body portion 56 and a second, free end 62 opposite the first end 61. The second arm 64 may have a first end 65. or base, attached to the body portion 56 and a second, free end 66 opposite the first end 65. In some embodiments, first and second arms 64 may diverge away from the body portion 56 at a convergence point at the base of first and second arms 60, 64. In the example illustrated in Figure 3, the body portion 56 and first and second arms 60, 64 define a Y shape. In other examples, the frame 46 may define a U shape. Alternatively, the second ends of the arms may be joined, forming a triangle, other polygon, circle, oval, or any desired shape. In some examples, the first and second arms 60, 64 may each have a fixed length and be fixed to the body portion 56. Arms 60. 64 and body portion 56 may be formed as a single, monolithic structure, or arms 60, 64 may be formed separate from body portion 56 and thereafter be joined to the body portion 56, if desired. In some instances, the body portion 56 may include an aperture 63. As will be discussed in greater detail below, aperture 63 may be utilized to attach the frame 46 to the delivery shaft 44.

In some examples (such as that shown in Figure 3), first and second arms 60, 64 may form a monolithic structure with body portion 56. In other words, in some examples body portion 56, first arm 60. and second arm 64 may be formed (e.g., machined, cut, shaped, stamped, laser-cut, etc.) from a single piece of material. In some examples, the frame 46 may be made of a superelastic and/or shape memory material such as nitinol. However, the above discussion is not intended to be limiting. Rather, it is contemplated that frame 46 may be constructed using alternative materials and/or manufacturing methodologies. For example, frame 46, or portions thereof, may be constructed from a polymeric material, a ceramic material and/or other various materials. Additionally, frame 46 may be manufactured via an injection molding or alternative polymer manufacturing methodologies. Alternatively, frame 46 may be formed through a 3-D printing process, if desired. Further, different portions of frame 46 (as described above, for example), may be made from a variety of materials and combined using alternative methodologies. For example, first and second arms 60, 64 may be made from a polymer material and combined with a body portion 56 constructed from a metal. Variations of combining different materials with different portions of frame 46 are contemplated.

The frame 46 may include a plurality of attachment members configured to releasably couple with the implant 12. The first arm 60 may have a first tab 70 disposed adjacent its free end 62, the second arm 64 may have a second tab 72 disposed adjacent its free end 66, and the body portion 56 having a third tab 74. In some instances, first arm 60 may include a plurality of first tabs 70 arranged along a length of the first arm 60 and/or second arm 64 may include a plurality of second arms 72 arranged along a length of the second arm 64. In some examples, the first, second, and third tabs 70, 72, 74 may include barbs or projections having a sharp tip configured to pierce and extend at least partially through the implant 12. In some instances the first, second and/or third tab 70, 72, 74 may extend entirely through the implant 12 to be exposed on a lower surface of the implant 12. However, in other instances the first, second and/or third tab 70, 72, 74 may extend only partially through the thickness of the implant 12, and thus not be exposed on the lower surface of the implant 12.

In the example illustrated in Figure 3, the first, second, and third tabs 70, 72, 74 are defined by cut-out portions of the frame 46. Thus, the arms 60, 64 and/or base 56 may include openings where the tabs 70, 72, 74 are cut from the material forming the arms 60 64 and/or base 56 when the tabs 70, 72, 74 are bent out of plane from the arms 60, 64 and/or base 56. In other examples, the first, second, and third tabs 70, 72, 74 may be formed separately and fixed to the frame 46. The first, second, and third tabs 70, 72, 74 may extend in different directions. As shown in Figure 3, the free end (e.g.. pointed end) of the first tab 70 extends toward the free end 62 of the first arm 60 and thus toward the distal end of the implant 12, the free end (e.g., pointed end) of the second tab 72 extends toward the body portion 56 and thus toward the proximal end of the implant 12, and the free end (e.g., pointed end) of the third tab 74 extends toward the free ends 62, 66 of the first and second arms 60, 64 and thus toward the distal end of the implant 12. Thus, the first tab 70 and the second tab 72 may be arranged to extend in generally opposite directions and/or may be arranged to extend toward opposite ends of the implant 12. In embodiments in which the first arm 60 includes a plurality of first tabs 70, each of the first tabs 70 may extend toward the free end 62 of the first arm and thus toward the distal end of the implant 12, if desired. In embodiments in which the second arm 64 includes a plurality of second tabs 72, each of the second tabs 72 may extend toward the body portion 56 and thus toward the proximal end of the implant 12, if desired.

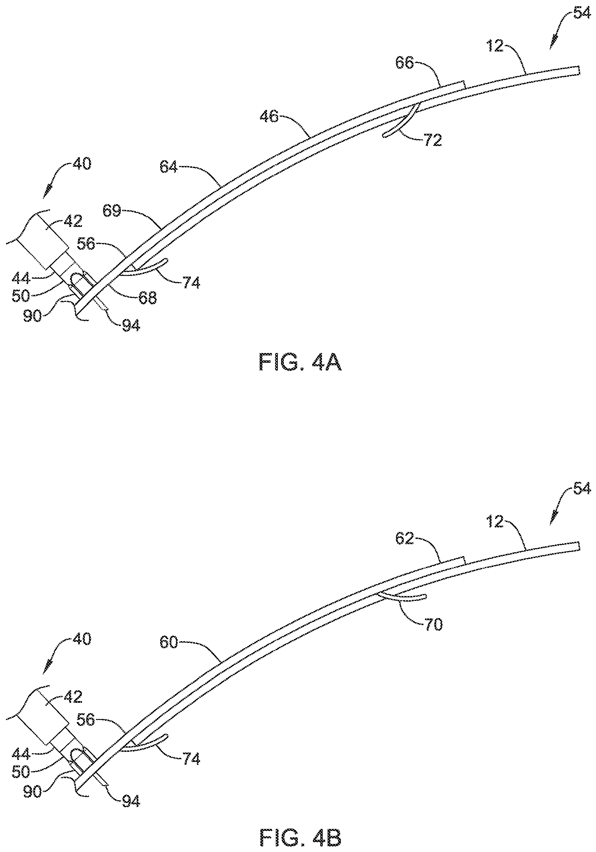

In the example shown in Figure 3, the third tab 74 has a rounded or blunt end and does not pierce the implant 12. Instead, the tab 74 is configured to grasp, wrap around or otherwise engage the proximal edge 17 of the implant 12. In other examples, the third tab 74 may have a sharp tip piercing and extending at least partially through the implant 12. The first and second arms 60, 64 may extend at an angle from the body portion 56 such that when the third tab 74 engages the edge 17 of the implant, the free ends of the first and second arms 60, 64 are disposed adjacent opposing edges of the implant 12. In some instances, first and second tabs 70, 72 may extend through implant 12 from the first side of implant 12 to the second side of implant 12. As illustrated, the first, second, and third tabs 70, 72, 74 extend downward from the lower surface 68 of the frame 46 which is juxtaposed with an upper surface of the implant 12. The upper surface 69 of the frame 46 may be devoid of any raised structures. The first, second, and third tabs 70, 72, 74 may be angled at an oblique angle relative to the frame 46, as illustrated in Figures 4A and 4B, in order to secure the implant 12 to the frame 46.

In some instances, one or more of the first, second, and third tabs 70, 72, 74 may be made of a superelastic and/or shape memory material, such as nitinol, configured to provide a secure grip on the implant 12 during delivery, but to be pulled free of the implant 12 without damaging the implant 12 as a withdrawal force is applied to frame 46. While a single tab is shown on each of the first and second arms 60, 64, it is contemplated that two or more tabs may be disposed on each arm. When multiple tabs are present on a single arm, the tabs may extend in the same direction or they may extend in opposite directions. In other words, in some instances, the first arm 60 may include one or more tabs having a free end (e.g., pointed end) extending toward the proximal end of the implant 12 and one or more tabs having a free end (e.g., pointed end) extending toward the distal end of the implant 12. Likewise, the second arm 64 may include one or more tabs having a free end (e.g., pointed end) extending toward the proximal end of the implant 12 and one or more tabs having a free end (e.g., pointed end) extending toward the distal end of the implant 12.

As stated above, it is contemplated in the examples discussed herein that frame 46 may be able to be detached from implant 12 upon securement of the implant 12 at a treatment site. For example, frame 46 may be configured to detach from implant 12 after implant 12 has been affixed to a target site in the body, such as with staples and/or sutures. Therefore, it can be appreciated that in some examples disclosed herein, frame 46 may be temporarily attached to implant 12. For example, frame 46 may be coupled, affixed or attached to implant 12 while positioned within delivery sheath 42, deployed out of delivery sheath 42 and maneuvered into position relative to a target site. Once positioned at the target site (e.g., along the tendon and/or humeral head), implant 12 may be securely affixed to the target site, such as stapled and/or sutured to bone and/or tendon tissue at the target site. However, once implant 12 has been securely affixed to the target site, frame 46 may be pulled away (e.g., detached) from implant 12 and removed from the body. The first and second tabs 70, 72 may be configured to be detached or disengaged from implant 12 upon application of a threshold level of force. For example, the second tab 72 shown in Figure 4A may provide frame 46 the ability to detach from implant 12 when a threshold "pull-away force" is applied to frame 46. For example, after implant 12 is affixed to a target site, a clinician may apply a force to frame 46 such that frame 46 is pulled away from implant 12. Provided the force is great enough (e.g., the threshold force is met), the first tab 70 and second tab 72 may be pulled back through the thickness of implant 12 as the third tab 74 disengages the edge 17 of the implant 12, thereby releasing frame 46 from implant 12.

Figure 4A shows the frame 46 coupled to the implant 12 via second and third tabs 72, 74 as described above. Further, Figure 4A shows frame 46 in combination with implant 12 coupled to an implant delivery shaft 44 extending through a delivery sheath 42. In some instances, delivery shaft 44 may be attached to the body portion 56 of frame 46 via a first connection member 90. The first connection member 90 may attach to body portion 56 via the aperture 63 (shown in Figure 3). In some instances, the first connection member 90 may be attached to the body portion 56 of frame 46 via a variety of mechanical fastening means (e.g., injection molding, encapsulation, bonding, etc.)

As discussed above, in some instances, a physician may insert implant delivery system 40 (including a delivery sheath 42, delivery shaft 44, frame 46 and implant 12) through an incision and position the distal end of the implant delivery system 40 adjacent a target implant site (e.g., torn tendon). Once adjacent the target site, the physician may manipulate the implant delivery shaft 44 to advance the implant (while attached to the detachable frame 46) out of the delivery sheath 42 adjacent the target implant site. For example, the physician may retract delivery sheath 42 proximally relative to delivery shaft 44 and frame 46 and/or may advance delivery shaft 44 and frame 46 distally relative to delivery sheath 42.

Figure 4A shows frame 46 and implant 12 deployed from the distal end of delivery sheath 42. In some instances, frame 46 may have a substantially concave shape with respect to delivery sheath 42. The concave curve may extend between the body portion 56 and the free ends 62, 66 of the first and second arms 60, 64. It can be appreciated that the concave shape of frame 46 may facilitate positioning the implant 12 along the generally rounded shape of the human shoulder, as shown in Figure 2.

However, when positioned in the delivery sheath 42 (e.g., prior to deployment) the frame 46 and implant 12 may be wrapped around the delivery shaft 44 in a convex configuration. Therefore, frame 46 and implant 12 may shift from a first convex configuration (while wrapped tightly around delivery shaft 44 within the lumen of delivery sheath 42) to a second concave configuration when advanced (e.g., deployed) out of delivery sheath 42.

In other words, frame 46 and implant 12 may be attached to the delivery shaft 44 when positioned within the lumen of the delivery sheath 42. In one example, when positioned within the delivery sheath 42, the frame 46 and implant 12 may wrap, or extend around, the delivery shaft 44. The position of the frame 46 and implant 12 may be in a convex, or rolled up configuration with respect to the distal portion 50 of the delivery shaft 44. As the frame 46 and implant 12 are deployed out of the distal end of the delivery sheath 42, the frame 46 and implant 12 may "shift" from a convex configuration to a concave configuration (as viewed with respect to the distal portion 50 of delivery shaft 44). Additionally, Figure 4A shows a tack member 94 extending through a portion of frame 46 such that the distal tip of tack member 94 would penetrate the head 14 of the humerus 16. In some examples, the tack member 94 may extend through the aperture 63 in the body portion 56 (see Figure 3), and/or extend from a connection member secured to the aperture 63. Tack member 94 will be described in greater detail below.

In some instances, the configuration of frame 46 shown in Figures 3, 4A, and 4B may provide both precise control and maneuverability to a clinician or other operator of the medical device. The delivery shaft 44 may be able to impart a downward force (e.g., a force directed toward a patient's shoulder) and/or rotational force onto frame 46 via the first connection member 90. Further, the concave geometry of frame 46 may allow the distal portion 54 of frame 46 to extend along the surface of the shoulder for which the implant 12 is to be positioned. In other words, the geometry of frame 46 shown in Figures 4A and 4B may prevent the distal portion 54 of frame 46 (including free ends 62, 66 of arms 60, 64) from pulling up and away from the shoulder surface as a clinician manipulates frame 46 within the body. Further, the geometry of frame 46 may allow the distal portion 54 of frame 46 to be advanced toward the surface of the shoulder in which an implant 12 is to be positioned.

As briefly described above, any of the implant delivery systems described herein may include a tack member 94 designed to anchor the delivery system in place prior to a clinician affixing implant 12 to the bone and/or tendon. As shown in Figure 4A. a tack member 94 extends distally from the first connection member 90. The tack member 94 may extend distally from the first connection member 90 and be substantially perpendicular to implant 12 and/or frame 46. The tack member 94 may be aligned along the central longitudinal axis of the delivery shaft 44. In some instances, tack member 94 may resemble a cylindrical pin or rod extending away from frame 46. The tack member 94 may be designed to be rigid enough to be pounded and/or inserted into bone. For example, in some instances, a clinician may apply a force to a proximal portion of the implant delivery system 40 (e.g., delivery shaft 44) such that tack member 94 may be "hammered" into a body structure (e.g., bone). In some instances, tack member 94 may include a tapered distal tip, which may be a sharpened or blunt tapered distal tip in some instances. The tack member 94 may include one or more flanges proximal of the distal tip. In some instances, tack member 94 may be stationary (e.g., fixed in place) relative to frame 46 and/or first connection member 90. In other instances, the tack member 94 may be retractable. The tack member 94 may be fixed to the frame 46 or may be removably attached to the frame 46.

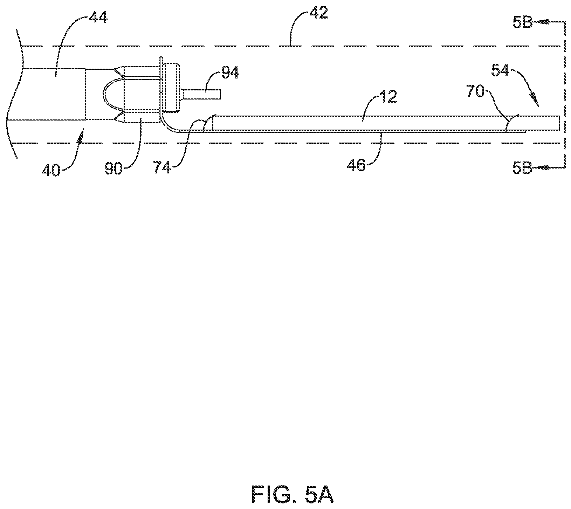

In some examples, frame 46 and implant 12 may be positioned within delivery sheath 42 (depicted as dashed line) as shown in Figure 5A. Figure 5A shows frame 46 (with implant 12) substantially aligned longitudinally with delivery shaft 44 and tack member 94. In this example, the distal portion 54 of frame 46 and implant 12 may be located distal of tack member 94 within delivery sheath 42. and thus are the first portion of delivery system 40 that exits the distal end 48 of delivery sheath 42 when the frame 46 and delivery shaft 44 are advanced out of the delivery sheath 42 upon deployment of the delivery system 40. In some examples, the frame 46 may be constrained into a rolled up shape within the lumen of the delivery sheath 42, however upon being advanced out of the delivery sheath 42, the frame 46 may resume its biased, curved shape, as shown in Figures 4A and 4B.

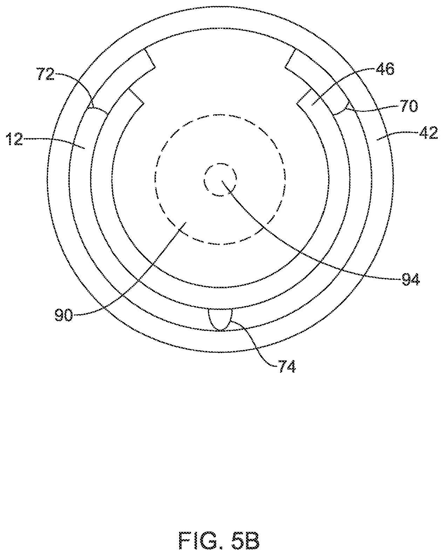

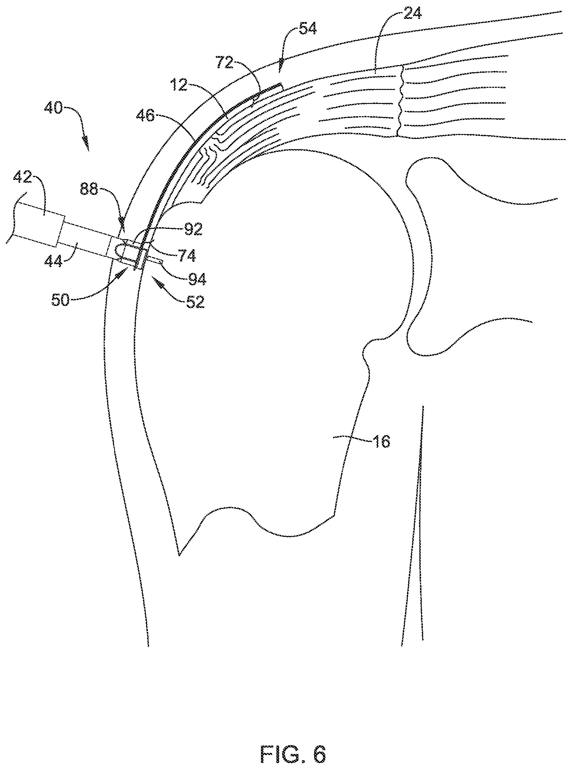

Additionally, Figure 5B illustrates that implant 12 may be rolled up and positioned between frame 46 and the delivery sheath 42. Upon exiting the distal end of delivery sheath 42, implant 12 may unwrap to a configuration illustrated in Figure 3 and Figures 4A and 4B. Figure 6 illustrates delivery system 40 with attached frame 46 and implant 12 after being maneuvered and/or positioned adjacent an example target site. The proximal portion 52 of the frame 46 is positioned adjacent the humeral head 16 with the tack member 94 driven into the humeral head 16. In this position, the distal portion 54 of the frame 46 is positioned adjacent the tendon 24, and the implant 12 is positioned over the tear 30 in the tendon 24.

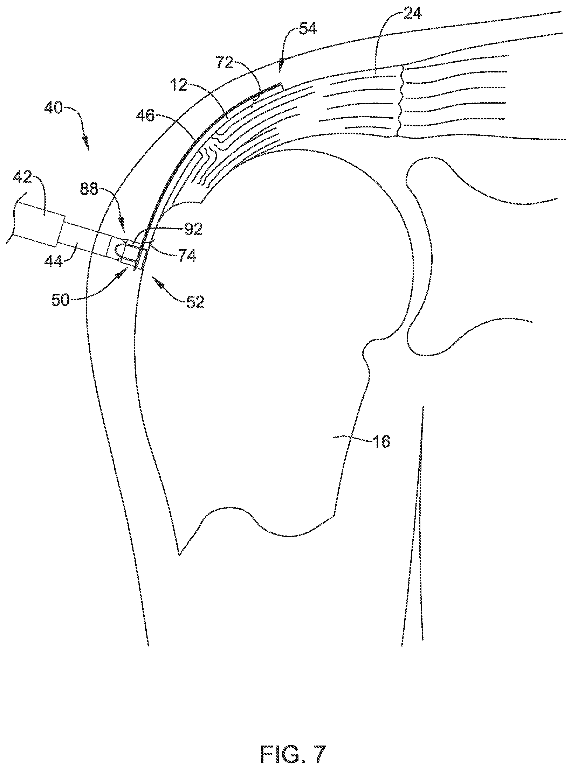

In other examples, tack member 94 may translate (e.g., slide, move, etc.) along a longitudinal axis within a lumen (not shown) of first connection member 90 of connection assembly 88. For example, Figure 7 shows example delivery system 40 positioned adjacent an example target site with the proximal portion 52 of the frame 46 (along with implant 12) positioned adjacent the humeral head 16. In this position, the distal portion 54 of the frame 46 is positioned adjacent the tendon 24. Figure 7 further illustrates that the tack member 94 has not been advanced and/or extended out of the first connection member 90 of connection assembly 88 and driven into the humeral head. Rather, the tack member 94 remains positioned within the connection assembly 88 (e.g., positioned within first connection member 90). However, in some examples contemplated herein, tack member 94 may be advanced out of the distal portion of delivery shaft 44 and/or connection assembly 88. In other words, the tack member 94 translates (e.g., slides, moves, etc.) relative to connection assembly 88 and advances away from the distal portion 50 of delivery shaft 44. Once the delivery shaft 44 (along with frame 46 and implant 12) has been maneuvered and/or positioned adjacent an example target site, as shown in Figure 7, the tack member 94 may be advanced out of the distal portion 50 of delivery shaft 44. Once the tack member 94 has been advanced out of the distal portion 50 of the delivery shaft 44 (e.g., advanced distally of first connection member 90) and into the humeral head 16. the device is in the position as shown in Figure 6. The tack member 94 may be advanced out of the distal portion 50 of delivery shaft 44 via the application of a force at the proximal end of the delivery system 40 and/or actuation of an actuation mechanism to move tack member 94 relative to first connection member 90. In some instances, a handle component may be utilized to generate a force to advance tack member 94 along a longitudinal axis of delivery shaft 44 and exit the distal portion 50 of delivery shaft 44 distal of first connection member 90.

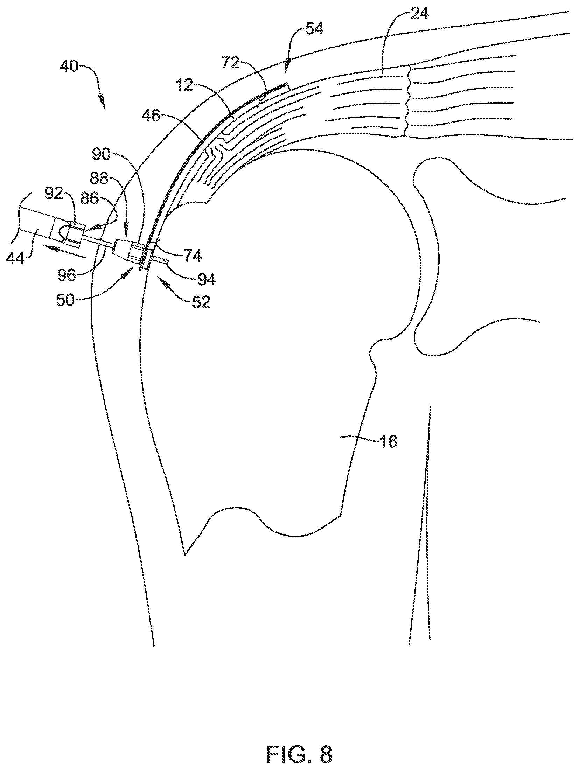

In some instances, once tack member 94 has been anchored into a target site (e.g.. humeral head 16) it may be desirable to remove the delivery shaft 44 to make room for additional instruments to be advanced adjacent the target site. Figure 8 illustrates removing delivery shaft 44 from the target site (depicted by the arrow in Figure 8) while the frame 46 and implant 12 remain anchored to the humeral head 16 via the tack member 94. As discussed above, delivery shaft 44 may be detached from frame 46 via uncoupling (e.g., detaching) a second connection member 92 from the first connection member 90. In some instances, it may be desirable to reengage delivery shaft 44 after detaching second connection member 92 from first connection member 90. For example, in some instances, the bone (e.g., humeral head) in which tack member 94 is initially inserted may be abnormally soft or hard, and therefore, may require additional force to either maintain placement (e.g., if the bone is too soft) or to remove (e.g., if the bone is too hard). Therefore, a clinician may choose to reinsert and reengage delivery shaft 44 to frame 46 via re-coupling second connection member 92 to first connection member 90, such as after implant 12 has been attached to a target site via one or more bone and/or tendon staples, as described below. Alternatively, delivery shaft 44 may remain engaged to frame 46 while attaching implant 12 to a target site via one or more bone and/or tendon staples, as described below. The clinician may then be able to apply additional force to frame 46 and/or tack member 94 when attaching implant 12 to an example target site via one or more bone and/or tendon staples.

In some instances, delivery system 40 may include a tether 96 coupled to frame 46. For example, Figure 8 shows tether 96 attached to first connection member 90. However, it is contemplated that in some examples tether 96 may be coupled directly to frame 46 and/or any other suitable structure. Further, tether 96 may be a rigid structure (e.g.. rod) or it may be a non-rigid structure (e.g., a wire, cable, suture, etc.). Additionally, it can be appreciated that tether 96 may be long enough to extend from frame 46 positioned at the target site to a location exterior of the patient through insertion site (i.e., incision), such as through a lumen 86 of delivery shaft 44 and out of a proximal portion of the implant delivery system 40 (e.g., proximal portion of delivery shaft 44). The tether 96 may be fixedly attached to the frame 46 such that the tether remains attached to the frame when the delivery shaft 44 is detached from the frame 46.

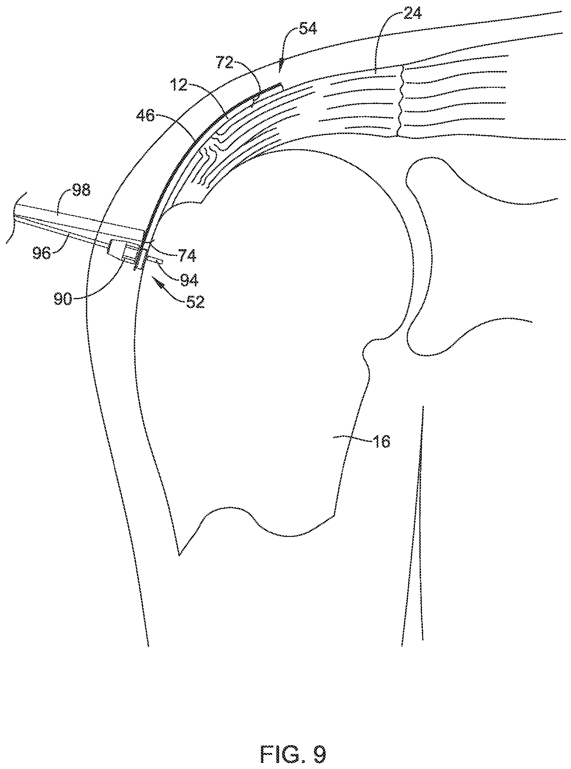

Further, it can be appreciated that tether 96 may remain attached to frame 46 (e.g., via first connection member 90) and extend to a location exterior of the patient through insertion site (i.e., incision) with delivery shaft 44 detached from frame 46 and removed from insertion site (i.e., incision) while additional instruments are advanced through the insertion site and to the target site. For example, Figure 9 shows a medical instrument 98 (e.g., implant stapler) positioned adjacent the proximal portion 52 of the frame 46 and implant 12. As discussed above and shown in Figure 8, tether 96 remains attached to frame 46 (e.g., via first connection member 90) and is positioned exterior of and alongside example medical instrument 98. The medical instrument 98 may be used to attach implant 12 to treatment site, such as with one or more, or a plurality of staples and/or sutures.

As discussed above, in some instances, implant 12 may be affixed to a target site after which the frame 46 may be detached (and removed) from both implant 12 and the target site. For example, in some instances, implant 12 may be attached to a target site via one or more bone and/or tendon staples. The staples may be applied to the target site via a stapling instrument (e.g., medical instrument 98).

Further, in some instances, it may be beneficial to affix implant 12 to the bone portion of the target site (e.g., humeral head 16) prior to affixing the implant to the tendon portion 24 of the target site. For example, it may be beneficial for a clinician to orient and/or position the frame 46 and implant 12 in the location/arrangement shown in Figure 9 prior to affixing the implant to the target site. As shown in Figure 9 (and previously discussed) the implant 12 is positioned such that the proximal portion 52 of the frame 46 and implant 12 are positioned adjacent the humeral head 16, while the distal portion 54 is positioned adjacent the tendon 24. Once the frame 46 and implant 12 have been placed appropriately, it may be desirable to utilize a stapling instrument to first insert staples along the proximal portion 52 of the implant 12 (e.g., the portion of the implant 12 positioned adjacent the bone) and into bone, followed by insertion of staples along the sides and distal portion of implant 12 and into tendon tissue.

It can be further appreciated that because the examples disclosed herein allow for the removal of the delivery sheath 42 and delivery shaft 44 prior to insertion of the stapling instrument, sufficient room exists to manipulate the stapling instrument in order to accurately place the staples along the proximal portion 52 of the implant 12 adjacent the humeral head 16. Further, as discussed above, the frame 46 is shaped to allow access to fastening regions 15 (shown in Figure 3) of the implant 12 so the implant 12 may be stapled without removing the frame 46.

Additionally, as discussed above, the tack member 94 may anchor the frame 46 and implant 12 in place (e.g., to the bone 16), thereby allowing a clinician to remove the delivery shaft 44 without fear that the frame/implant 46/12 combination will change position prior to the insertion of staples into the implant 12.

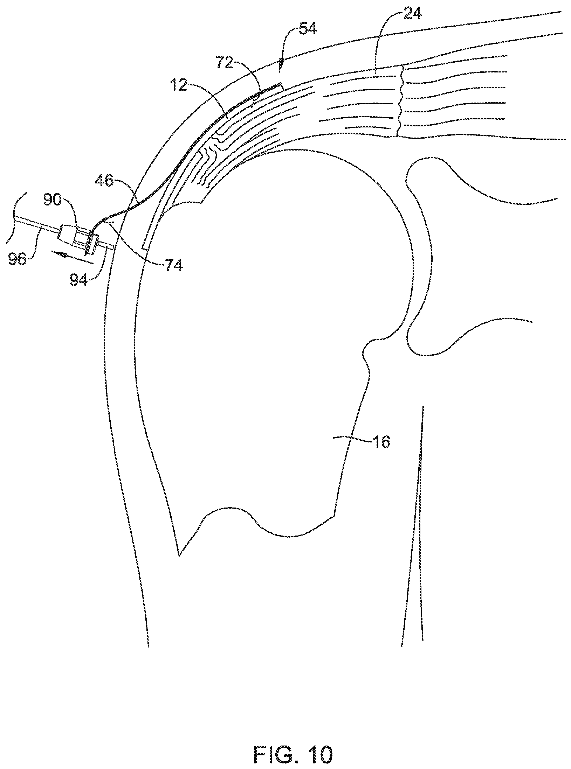

Once the implant 12 has been sufficiently affixed to the target site, the clinician may detach the frame 46 from the implant 12 (within the body) and remove it from the body via the insertion site. For example, Figure 10 shows the detachment and removal of the frame 46 from the implant 12 (within the body) after the implant 12 has been affixed (e.g., via staples) to the target site. In some instances, the clinician may detach and remove frame 46 from the implant 12 and the body via application of a withdrawal force to the frame 46. The withdrawal force may be applied via the tether 96. For example, a clinician may pull on the tether 96 (the proximal end of which may be positioned outside of the body), thereby applying a withdrawal force to frame 46. Once the withdrawal force reaches a threshold level (as discussed above), the first, second, and third tabs 70, 72, 74 on the frame 46 will detach from implant 12. Further withdrawal of the tether 96 may be continued to pull frame 46 out of the body via the insertion site.

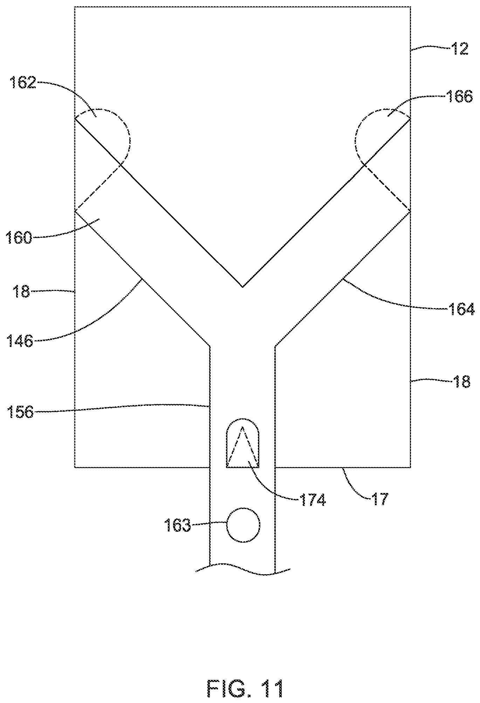

Figure 11 illustrates another example frame 146 for releasably holding a sheet-like or planar implant 12. The frame 146 is similar to the frame 46 shown in Figure 3, except that instead of first and second tabs 70, 72 on arms 60, 64 securing the implant 12, as in Figure 3, the free end 162 on the first arm 160 and the free end 166 on the second arm 164 of the frame 146 are bent to define first and second tabs configured to removably attached to the implant 12 by wrapping around edges (e.g. wrapping around opposing side edges 18) of the implant 12. When the first and second arms 160, 164 are disposed on and juxtaposed with an upper surface of a planar implant 12, the free ends 162, 166 of the first and second arms 160, 164 wrap around side edges 18 of the implant 12 and extend along and face a lower surface of the implant 12. The implant 12 may be grasped between the free ends 162, 166 (on the lower surface of the implant 12) and the medial portion of the first and second arms 160, 164 (on the upper surface of the implant 12).

The implant 12 shown in Figure 11 is rectangular, with the free ends 162, 166 wrapping around and clamping the opposing side edges 18 of the implant 12. Similar to the frame 46 shown in Figure 3, frame 146 has a tab 174 disposed on the body portion 156 of the frame 146 that is configured to grasp or be juxtaposed with lower edge 17 of the implant 12. When the tab 174 engages one edge 17 of the implant 12, the free ends 162, 166 of the first and second arms 160, 164 are disposed adjacent opposing side edges 18 of the implant 12. For example, when the implant 12 is a polygon and the tab 174 engages a first edge 17 of the implant 12, the first and second free ends 162, 166 of the first and second arms 160, 164 are configured to engage (e.g., wrap around) second and third opposing side edges 18 of the implant 12. In instances in which the implant 12 is circular and the tab 174 may engage a first location on the edge of the implant 12, the first and second free ends 162, 166 of the first and second arms 160, 164 are configured to engage (e.g., wrap around) second and third locations on the edge of the implant 12 spaced apart from the first location.

The frame 146, coupled to the implant 12, may be secured to a delivery system 40 including a delivery shaft 44, as described above. The free ends 162, 166 and tab 174 combine to securely hold the implant 12 during delivery, yet allow for the frame 146 to be pulled free of the implant 12 after the implant 12 has been stapled or otherwise secured within the body.

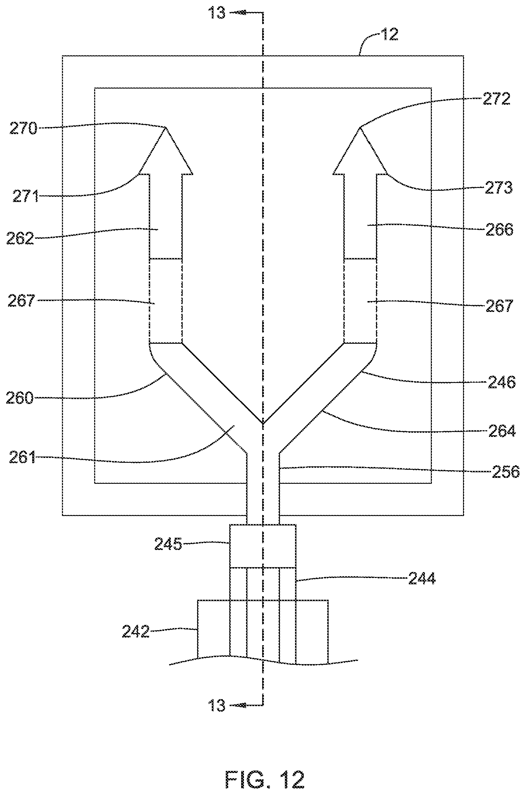

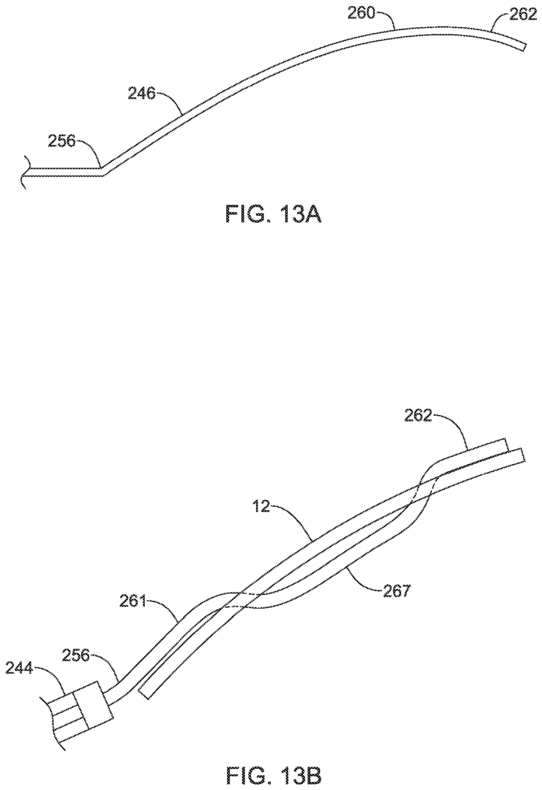

Another example frame 246 for delivering the implant 12 is illustrated in Figure 12. The frame 246 is configured to be releasably attached to the implant 12. The frame 246 may include a body portion 256, a first arm 260 and a second arm 264 extending from the body portion 256. The body portion 256 may be positionable along a central portion of the implant 12 with first and second arms 260, 264 extending laterally from the body portion 256 in opposite directions toward opposite side edges of the implant 12. In some examples, the first and second arms 260, 264 have free ends 262, 266 including a sharp distal tip 270, 272 and a flared region 271, 273 proximal of the sharp distal tip 270, 272 configured to penetrate the implant 12 when inserted through the implant 12 in a first direction, and to resist withdrawal from the implant 12 when pulled in a second direction opposite the first direction. The flared region 271, 273 may have a width larger than a width of the arm 260. 264 immediately proximal of the flared region 271, 273 to define a shoulder portion. The first and second arms 260, 264 may be woven into and out of the implant 12, as shown in Figure 12. The first and second arms 260, 264 may be fixed to the body portion 256 and may have a fixed length. In some examples, the entire frame 246, including the body portion 256 and first and second arms 260, 264, is formed from as a single, monolithic piece. The first and second arms 260, 264 may have a fixed length. The first and second arms 260, 264 may diverge from the body portion 256. For example, the body portion 256 and first and second arms 260, 264 may define a Y shape, as shown in Figure 12. In some examples, the frame 246 may define a single concave arc between the body portion 256 and the free ends 262, 266 of the first and second arms 260. 264, as shown in Figure 13A. One example of the woven configuration of the first and second arms 260, 264 into and out of the implant 12 is illustrated in Figure 13B. In some examples, the body portion 256 and first end 261 and free end 262 of each arm 260 lies along an upper surface of the implant 12 while a middle region 267 of each arm 260 lies along a lower surface of the implant 12, as shown in Figure 13B.

The frame 246 illustrated in Figure 12 is in the deployed state with the first and second arms 260, 264 extending at an oblique angle from the body portion 256. The frame 246 may be attached to a delivery system including a delivery sheath 242 having a proximal end, a distal end, and a lumen extending therebetween, and a delivery shaft 244 extending within the lumen of delivery sheath 242 and longitudinally movable relative thereto. The frame 246 may be rigidly and fixedly attached to the distal end 245 of the delivery shaft 244. In some examples, the implant 12 is deployed substantially in-line with the delivery shaft 244. In other examples, the frame 246 may have a curve that matches a curved target site. Additionally, in some examples the proximal portion of delivery shaft 444 and/or or delivery sheath 242 may be coupled to a handle member (not shown). The handle member may be utilized to manipulate delivery shaft 244. For example, the handle member may be utilized to impart a rotational and/or longitudinal force to delivery shaft 244 during deployment.

In the undeployed state, frame 246 may be configured to fit within the delivery sheath 242. Accordingly, in such an example, each arm 260, 264 may deform in a manner to allow insertion of the frame 246 and attached implant 12 into delivery sheath 242. Accordingly, the frame 246 may generally be flexible, and in the example of Figure 12, each arm 260, 264 may bend, twist, fold, wrap or otherwise deform in order for the frame 246 to fit within the delivery sheath 242, and to return to the deployed shape shown in Figure 12 when extended out of the delivery sheath 242.

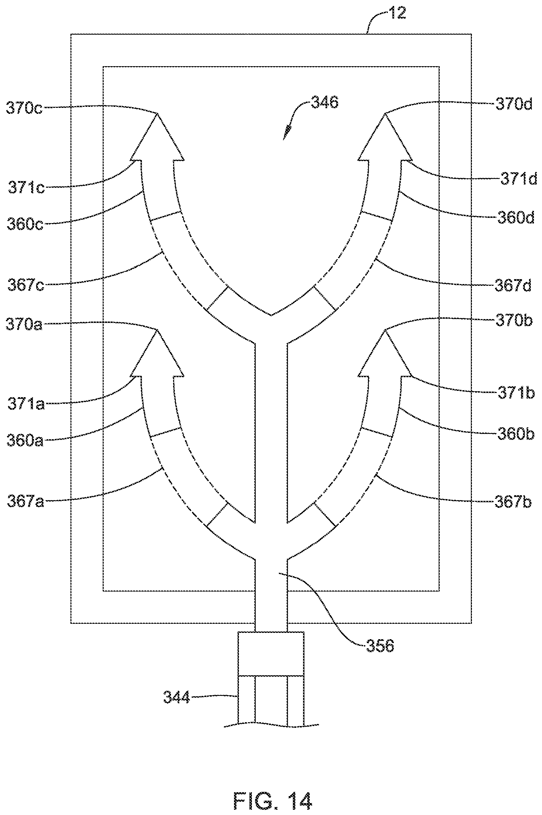

Although Figure 12 depicts the frame 246 having a Y shape, it will be understood that the frame 246 can be shaped in any number of different configurations. For instance, as illustrated in Figure 14, the frame 346 includes multiple sets of arms that diverge from the main body 356. The main body 356 may extend in a longitudinal direction from the inner shaft 344 and a plurality of appendages that extend away from the trunk at an angle. Figure 14 depicts frame 346 with a main body 356 and four arms 360a, 360b. 360c, 360d (collectively 360). The main body 356 may be positioned or juxtaposed along an upper surface of the implant 12. The arms 360 may have a base attached to the main body 356 and also positioned or juxtaposed along the upper surface of the implant 12. The arms 360 may have a free end ending in a sharp distal tip 370a, 370b, 370c, 370d (collectively 370) and a flared region 371a, 371b, 371c, 371d (collectively 371) proximal of the sharp distal tip 370. The sharp distal tip 370 may be configured to penetrate the implant 12 when inserted through the implant 12 in a first direction, and the flared region 371 may resist withdrawal from the implant 12 when pulled in a second direction opposite the first direction. The arms 360 may be woven into and out of the implant, as shown in Figure 14, with a medial portion 367a, 367b, 367c, 367d (collectively 367) of each arm 360 facing or juxtaposed with the lower surface of the implant 12, opposite the upper surface. The distal tips 370 of the arms 360 may be positioned on and face the upper surface of the implant 12.

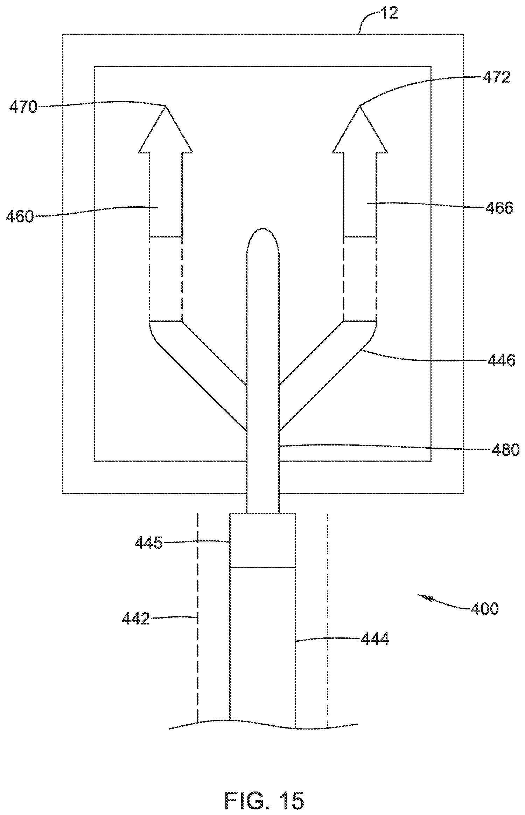

Figure 15 illustrates another example of an implant delivery system 400 that may be used to deliver an implant 12 to a target site in the body. The implant delivery system 400 may be substantially similar to the implant delivery system disclosed in U.S. Patent No. 10,258,459. The implant delivery system 400 may include a delivery sheath 442 and a delivery shaft 444 slidable within a lumen of the delivery sheath 442. In some examples, first and second beams 480 may be fixed to the distal end 445 of the delivery shaft 444 and extending substantially parallel to the delivery shaft 444. In the top view shown in Figure 15, only the first beam 480 is shown extending over the implant 12. The second beam mirrors the first beam 480 and extends below the implant 12 directly under the first beam 480. The implant 12 may be disposed between the first and second beams 480, and first and second beams 480 may releasably retain or grasp implant 12. For instance, first and second beams 480 may passively retain implant 12 when implant 12 is positioned between first and second beams 480, such as by contact forces between first and second beams 480 and implant 12. Alternatively, first and second beams 480 may be biased towards each other, creating a gripping force to retain implant 12.

The implant delivery system 400 may include an active retention mechanism to retain implant 12. The active retention mechanism may require manipulation by a user to retain and/or release implant 12. In some examples, the active retention mechanism may include a frame 446 releasably connected to delivery shaft 444. In some examples, frame 446 may be fixed to the distal end 445 of delivery shaft 444 and extend substantially parallel to the delivery shaft 444. In other examples, frame 446 may be fixed to first beam 480, frame 446 may be incorporated with first beam 480, or frame 446 may be positioned between first and second beams 480. When implant 12 is disposed between first and second beams 480, frame 446 may engage implant 12. In other examples, the implant delivery system 400 does not include the first and second beams 480. Instead, frame 446 alone may releasably engage the implant 12.

The frame 446 may be similar to frame 246 described above, with first and second arms 460, 466 having sharp distal tips 470, 472 configured to penetrate implant 12 when inserted in a first direction and to resist withdrawal from implant 12 when pulled in a second direction opposite the first direction. The arms may be woven into and out of implant 12 as shown in Figure 15. In some examples, first and second beams 480 and frame 446 may be fixed to the distal end 445 of delivery shaft 444 such that implant 12 extends generally parallel to the longitudinal axis of delivery shaft 444.

In implant delivery system 400, once implant 12 is moved out of delivery sheath 442, implant 12 may extend generally parallel to the longitudinal axis of the delivery shaft 444, as illustrated in Figure 15. Implant delivery system 40 provides an alternative position for implant 12 in that once implant 12 is moved out of delivery sheath 42. implant 12 extends at an angle, such as substantially perpendicular) to the longitudinal axis of the delivery shaft 44, as illustrated in Figure 2. The frames 46. 146, 246, 346, 446 may be connected to either of the implant delivery systems 40, 400. depending on the desired angle/orientation/trajectory of delivery.

In the undeployed state, frame 46, 146, 246, 346, 446 may be configured to fit within delivery sheath 42, 242, 442. Accordingly, in such an example, each arm of frame 46, 146, 246, 346, 446 may deform in a manner to allow insertion of frame 46, 146, 246, 346, 446 into delivery sheath 42. 242, 442. Accordingly, frame 46, 146, 246, 346, 446 may generally be flexible, and each arm of frame 46, 146, 246, 346, 446 may bend, twist, fold, wrap or otherwise deform in order for frame 46. 146, 246, 346, 446 with attached implant 12 to fit within delivery sheath 42, 242, 442.

In at least some examples, the frame 46, 146, 246, 346, 446 is made of a material that may deform elastically into one or more shapes in order to fit within the confines of delivery sheath 42, 242, 442. Some suitable example materials include metals and metal alloys including stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N 10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like: cobalt-chromium alloys; cobalt-chromium-molybdenum alloys (e.g., UNS: R30003 such as ELGILOY^{®}, PHYNOX^{®}, and the like); platinum enriched stainless steel; titanium: combinations thereof; and the like; or any other suitable material.

As alluded to above, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some examples, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some examples, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In other examples, implant positioning component may be constructed of one or more of the above described materials configured as an inlay. For instance, the frame 46, 146, 246, 346, 446 may comprise a metal structure encased in one or more other materials, such as a plastic or silicone material. The plastic or silicone material may be molded either completely or partly over the metal structure. Such hybrid-material structures may reduce the manufacturing cost of producing frame 46, 146, 246, 346, 446 or provide frame 46, 146, 246, 346, 446 with physical properties unable to be achieved by using only metal.

In the above examples, implant 12 may comprise one or multiple of a number of different materials. In some examples, implant 12 may comprise a plurality of fibers. The fibers may be interlinked with one another. When this is the case, implant 12 may comprise a plurality of apertures comprising the interstitial spaces between fibers. Various processes may be used to interlink the fibers with one another. Examples of processes that may be suitable in some applications including weaving, knitting, and braiding. In some embodiments, implant 12 may comprise a laminate including multiple layers of film with each layer of film defining a plurality of micro-machined or formed holes. Implant 12 may also comprise a reconstituted collagen material having a porous structure. Additionally, implant 12 may also comprise a plurality of electro-spun nanofiber filaments forming a composite sheet. Additionally, implant 12 may comprise a synthetic sponge material that defines a plurality of pores. Implant 12 may also comprise a reticulated foam material. Implant 12 may be circular, oval, oblong, square, rectangular, triangular, or any other shape configured to suit the target anatomy.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments.

## Claims

1. An implant delivery system (40) for delivering an implant (12), comprising:
a delivery shaft (44) including a proximal portion and a distal portion (50);
a frame (46) coupled to the distal portion of the delivery shaft, the frame including a body portion (56) and at least first and second arms (60, 64), the first and second arms each having first ends (61, 65) attached to the body portion and second, free ends (62, 66) opposite the first ends, the first arm having a first tab (70) disposed adjacent its free end, the second arm having a second tab (72) disposed adjacent its free end, and the body portion having a third tab (74), the first, second, and third tabs each being defined by cut-out portions of the frame, wherein the first and second tabs include a sharp tip configured to extend at least partially through the implant, wherein the first tab extends toward the free end of the first arm and the second tab extends toward the body portion, and wherein the first, second, and third tabs are all configured to be releasably attached to the implant.

2. The implant delivery system of claim 1, wherein the body portion and first and second arms define a Y shape,
with the first and second arms each extending at an angle from the body portion such that when the third tab (74) engages an edge (17) of the implant (12), the free ends of the first and second arms are disposed adjacent opposing edges of the implant

3. The implant delivery system of claim 2, wherein the third tab extends toward the free ends of the first and second arms.

4. The implant delivery system of claim 1, wherein the frame (46) has an upper surface (69) and a lower surface (68), and the first and second tabs extend downward from the lower surface.

5. The implant delivery system of claim 1, wherein an entirety of the frame, including the body portion and first and second arms, is formed from a single, monolithic piece.

6. The implant delivery system of claim 1, wherein the frame (46) is removably coupled to the delivery shaft (44), and configured to be detached from the delivery shaft in vivo, the implant delivery system further comprising a tether (96) extending through a lumen of the delivery shaft, the tether fixedly attached to the frame such that the tether remains attached to the frame when the delivery shaft is detached from the frame.

7. An implant delivery system, comprising:
a delivery shaft including a proximal portion and a distal portion;
a frame (146) coupled to the distal portion of the delivery shaft, the frame including a body portion (156) and at least first and second arms (160, 164) extending from the body portion, the frame including a plurality of tabs configured to releasably engage an implant, the first and second arms each having free ends, the free end (162) of the first arm defining a first tab, the free end (166) of the second arm defining a second tab, and the body portion having a third tab (174), wherein the first, second, and third tabs are configured to engage at least one edge of the implant,
wherein the free ends of the first and second arms are bent such that when the first and second arms are disposed on an upper surface of a planar implant, the free ends of the first and second arms wrap around side edges of the implant forming first and second tabs that extend along a lower surface of the implant.

8. The implant delivery system of claim 7, wherein the body portion and first and second arms define a Y shape,
wherein the first and second arms each extend at an angle from the body portion such that when the third tab (174) engages the at least one edge (17) of the implant, the free ends of the first and second arms are disposed adjacent the at least one edge at opposing edges of the implant

9. The implant delivery system of claim 8, wherein when the implant is a polygon and the third tab engages a first edge of the implant, the first and second tabs of the first and second arms are configured to engage second and third edges of the implant adjacent the first edge of the implant

10. The implant delivery system of claim 8, wherein when the implant is circular and has an outer edge, and the third tab engages a first location on the outer edge, the first and second tabs of the first and second arms are configured to engage second and third locations on the outer edge of the implant spaced apart from the first location.

11. An implant assembly, comprising:
a one-piece planar implant (12); and
a delivery system including:
an outer shaft (242) including a proximal end, a distal end, and a lumen extending therebetween;
an inner shaft (244) slidably disposed within the lumen of the outer shaft; and
a frame (246) fixedly attached to a distal end of the inner shaft and configured to be releasably attached to the implant, the frame including a body portion (256) and at least first and second arms (260, 264) extending from the body portion, each of the first and second arms having a free end (262, 266) configured to penetrate the implant when inserted through the implant in a first direction, and to resist withdrawal from the implant when pulled in a second direction opposite the first direction;
wherein the first and second arms (260, 264) are woven into and out of the implant (12).

12. The implant assembly of claim 11, wherein the free ends of the first and second arms each include a sharp distal tip (270, 272) and a flared region (271, 273) proximal of the sharp distal tip.

13. The implant assembly of claim 11, wherein the frame, including the body portion and first and second arms, is formed from as a single, monolithic piece.

14. The implant assembly of claim 11, wherein the frame defines a single concave arc between the body portion (256) and the free ends (262, 266) of the first and second arms.

15. The implant assembly of claim 11, wherein the body portion and first and second arms define a Y shape.

## Patentansprüche

1. Implantateinführsystem (40) zum Einführen eines Implantats (12), aufweisend:
einen Einführschaft (44) mit einem proximalen Abschnitt und einem distalen Abschnitt (50);
einen Rahmen (46), der mit dem distalen Abschnitt des Einführschafts gekoppelt ist, wobei der Rahmen einen Körperabschnitt (56) und mindestens einen ersten und einen zweiten Arm (60, 64) aufweist, wobei der erste und der zweite Arm jeweils ein erstes Ende (61, 65), das an dem Körperabschnitt angebracht ist, und ein zweites, freies Ende (62, 66) gegenüber dem ersten Ende aufweisen, wobei der erste Arm eine erste Lasche (70) aufweist,
die neben seinem freien Ende angeordnet ist, wobei der zweite Arm eine zweite Lasche (72) aufweist, die neben seinem freien Ende angeordnet ist, und der Körperabschnitt eine dritte Lasche (74) aufweist, wobei die erste, die zweite und die dritte Lasche jeweils durch ausgeschnittene Abschnitte des Rahmens definiert sind, wobei die erste und die zweite Lasche eine scharfe Spitze aufweisen, die dazu konfiguriert ist, sich mindestens teilweise durch das Implantat hindurch zu erstrecken, wobei sich die erste Lasche zum freien Ende des ersten Arms erstreckt und sich die zweite Lasche zum Körperabschnitt erstreckt, und
wobei die erste, die zweite und die dritte Lasche dazu konfiguriert sind, lösbar an dem Implantat angebracht zu sein.

2. Implantateinführsystem nach Anspruch 1, wobei der Körperabschnitt und der erste und zweite Arm eine Y-Form definieren,
wobei sich der erste und der zweite Arm jeweils in einem solchen Winkel von dem Körperabschnitt erstrecken, dass die freien Enden des ersten und des zweiten Arms jeweils an gegenüberliegenden Kanten des Implantats angeordnet sind, wenn die dritte Lasche (74) mit einer Kante (17) des Implantats (12) in Eingriff steht.

3. Implantateinführsystem nach Anspruch 2, wobei sich die dritte Lasche zu den freien Enden des ersten und zweiten Arms erstreckt.

4. Implantateinführsystem nach Anspruch 1, wobei der Rahmen (46) eine Oberseite (69) und eine Unterseite (68) aufweist und sich die erste und die zweite Lasche von der Unterseite nach unten erstrecken.

5. Implantateinführsystem nach Anspruch 1, wobei der gesamte Rahmen einschließlich des Körperabschnitts und des ersten und zweiten Arms aus einem einzigen monolithischen Stück gebildet ist.

6. Implantateinführsystem nach Anspruch 1, wobei der Rahmen (46) lösbar mit dem Einführschaft (44) gekoppelt und dazu konfiguriert ist, in vivo vom Einführschaft gelöst zu werden, wobei das Implantateinführsystem ferner ein Halteseil (96) aufweist, das sich durch ein Lumen des Einführschafts erstreckt, wobei das Halteseil fest an dem Rahmen angebracht ist, sodass das Halteseil an dem Rahmen angebracht bleibt, wenn der Einführschaft vom Rahmen gelöst wird.

7. Implantateinführsystem, aufweisend:
einen Einführschaft mit einem proximalen Abschnitt und einem distalen Abschnitt;
einen Rahmen (146), der mit dem distalen Abschnitt des Einführschafts gekoppelt ist, wobei der Rahmen einen Körperabschnitt (156) und mindestens einen ersten und einen zweiten Arm (160, 164) aufweist, die sich von dem Körperabschnitt erstrecken, wobei der Rahmen mehrere Laschen aufweist, die dazu konfiguriert sind, ein Implantat lösbar in Eingriff zu nehmen, wobei der erste und der zweite Arm jeweils ein freies Ende aufweisen, wobei das freie Ende (162) des ersten Arms eine erste Lasche definiert, das freie Ende (166) des zweiten Arms eine zweite Lasche definiert und der Körperabschnitt eine dritte Lasche (174) aufweist, wobei die erste, zweite und dritte Lasche dazu konfiguriert sind, mindestens eine Kante des Implantats in Eingriff zu nehmen,
wobei die freien Enden des ersten und des zweiten Arms jeweils so gebogen sind, dass sich die freien Enden des ersten und des zweiten Arms um Seitenkanten des Implantats wickeln und eine erste und eine zweite Lasche bilden, die sich entlang einer Unterseite des Implantats erstrecken, wenn der erste und der zweite Arm auf einer Oberseite eines ebenen Implantats angeordnet sind.

8. Implantateinführsystem nach Anspruch 7, wobei der Körperabschnitt und der erste und zweite Arm eine Y-Form definieren,
wobei sich der erste und der zweite Arm jeweils in einem solchen Winkel von dem Körperabschnitt erstrecken, dass die freien Enden des ersten und des zweiten Arms neben der mindestens einen Kante an gegenüberliegenden Kanten des Implantats angeordnet sind, wenn die dritte Lasche (174) mit der mindestens einen Kante (17) des Implantats in Eingriff steht.

9. Implantateinführsystem nach Anspruch 8, wobei die erste und zweite Lasche des ersten und zweiten Arms dazu konfiguriert sind, in eine zweite und dritte Kante des Implantats neben der ersten Kante des Implantats einzugreifen, wenn das Implantat ein Polygon ist und die dritte Lasche in eine erste Kante des Implantats eingreift.

10. Implantateinführsystem nach Anspruch 8, wobei die erste und zweite Lasche des ersten und zweiten Arms, wenn das Implantat kreisförmig ist und eine Außenkante aufweist und die dritte Lasche in eine erste Stelle an der Außenkante eingreift, dazu konfiguriert sind, in eine zweite und dritte Stelle an der Außenkante des Implantats einzugreifen, die von der ersten Stelle beabstandet ist.

11. Implantatanordnung, aufweisend:
ein einstückiges, flächiges Implantat (12); und
ein Einführsystem, aufweisend:
einen Außenschaft (242), der einen proximalen Abschnitt, einen distalen Abschnitt und ein sich dazwischen erstreckendes Lumen aufweist;
einen Innenschaft (244), der verschiebbar im Lumen des Außenschafts angeordnet ist; und
einen Rahmen (246), der fest an einem distalen Ende des Innenschafts angebracht und dazu konfiguriert ist, lösbar an dem Implantat angebracht zu werden, wobei der Rahmen einen Körperabschnitt (256) und mindestens einen ersten und einen zweiten Arm (260, 264) aufweist, die sich von dem Körperabschnitt aus erstrecken, wobei der erste und der zweite Arm jeweils ein freies Ende (262, 266) aufweisen, das so konfiguriert ist, dass es in das Implantat eindringt, wenn es in einer ersten Richtung durch das Implantat eingeführt wird, und dass es dem Herausziehen aus dem Implantat widersteht, wenn es in einer zur ersten Richtung entgegengesetzten zweiten Richtung gezogen wird;
wobei der erste und der zweite Arm (260, 264) in das Implantat (12) eingewoben und aus ihm herausgewoben sind.

12. Implantatanordnung nach Anspruch 11, wobei die freien Enden des ersten und zweiten Arms jeweils eine scharfe distale Spitze (270, 272) und einen sich erweiternden Bereich (271, 273) proximal zu der scharfen distalen Spitze aufweisen.

13. Implantatanordnung nach Anspruch 11, wobei der Rahmen einschließlich des Körperabschnitts und des ersten und zweiten Arms aus einem einzigen, monolithischen Stück geformt ist.

14. Implantatanordnung nach Anspruch 11, wobei der Rahmen einen einzelnen konkaven Bogen zwischen dem Körperabschnitt (256) und den freien Enden (262, 266) des ersten und zweiten Arms definiert.

15. Implantatanordnung nach Anspruch 11, wobei der Körperabschnitt und der erste und zweite Arm eine Y-Form definieren.

## Revendications

1. Système de pose d'implant (40) pour poser un implant (12), comprenant :
une tige de pose (44) incluant une partie proximale et une partie distale (50) ;
une armature (46) couplée à la partie distale de la tige de pose, l'armature incluant une partie corps (56) et au moins des premier et deuxième bras (60, 64), les premier et deuxième bras ayant chacun des premières extrémités (61, 65) attachées à la partie corps et des deuxièmes extrémités libres (62, 66) opposées aux premières extrémités, le premier bras ayant une première languette (70) disposée de façon adjacente à son extrémité libre, le deuxième bras ayant une deuxième languette (72) disposée de façon adjacente à son extrémité libre et la partie corps ayant une troisième languette (74), les première, deuxième et troisième languettes étant chacune définie par des parties découpées de l'armature, dans lequel les première et deuxième languettes incluent une pointe acérée configurée pour s'étendre au moins partiellement à travers l'implant, dans lequel la première languette s'étend vers l'extrémité libre du premier bras et la deuxième languette s'étend vers la partie corps, et dans lequel les première, deuxième et troisième languettes sont toutes configurées pour être attachées de manière détachable à l'implant.

2. Système de pose d'implant selon la revendication 1, dans lequel la partie corps et les premier et deuxième bras définissent une forme en Y,
les premier et deuxième bras s'étendant chacun en formant un angle par rapport à la partie corps de telle sorte que lorsque la troisième languette (74) se met en prise avec un bord (17) de l'implant (12), les extrémités libres des premier et deuxième bras sont disposées de façon adjacente aux bords opposés de l'implant.

3. Système de pose d'implant selon la revendication 2, dans lequel la troisième languette s'étend vers les extrémités libres des premier et deuxième bras.

4. Système de pose d'implant selon la revendication 1, dans lequel l'armature (46) a une surface supérieure (69) et une surface inférieure (68), et les première et deuxième languettes s'étendent vers le bas par rapport à la surface inférieure.

5. Système de pose d'implant selon la revendication 1, dans lequel l'ensemble de l'armature, incluant la partie corps et les premier et deuxième bras, est formé à partir d'une seule pièce monolithique.

6. Système de pose d'implant selon la revendication 1, dans lequel l'armature (46) est couplée de manière amovible à la tige de pose (44), et configurée pour être détachée de la tige de pose in vivo, le système de pose d'implant comprenant en outre une attache (96) s'étendant à travers une lumière de la tige de pose, l'attache étant fixée solidement à l'armature de telle sorte que l'attache reste fixée à l'armature lorsque la tige de pose est détachée de l'armature.

7. Système de pose d'implant, comprenant :
une tige de pose incluant une partie proximale et une partie distale ;
une armature (146) couplée à la partie distale de la tige de pose, l'armature incluant une partie corps (156) et au moins des premier et deuxième bras (160, 164) s'étendant à partir de la partie corps, l'armature incluant une pluralité de languettes configurées pour se mettre en prise avec un implant de manière détachable, les premier et deuxième bras ayant chacun des extrémités libres, l'extrémité libre (162) du premier bras définissant une première languette, l'extrémité libre (166) du deuxième bras définissant une deuxième languette et la partie corps ayant une troisième languette (174), dans lequel les première, deuxième et troisièmes languettes sont configurées pour se mettre en prise avec au moins un bord de l'implant,
dans lequel les extrémités libres des premier et deuxième bras sont inclinées de telle sorte que lorsque les premier et deuxième bras sont disposés sur une surface supérieure d'un implant plan, les extrémités libres des premier et deuxième bras s'enroulent autour des bords latéraux de l'implant, formant les première et deuxième languettes qui s'étendent le long d'une surface inférieure de l'implant.

8. Système de pose d'implant selon la revendication 7, dans lequel la partie corps et les premier et deuxième bras définissent une forme en Y,
dans lequel les premier et deuxième bras s'étendent chacun en formant un angle par rapport à la partie corps de telle sorte que lorsque la troisième languette (174) se met en prise avec l'au moins un bord (17) de l'implant, les extrémités libres des premier et deuxième bras sont disposées de façon adjacente à l'au moins un bord au niveau de bords opposés de l'implant.

9. Système de pose d'implant selon la revendication 8, dans lequel lorsque l'implant est un polygone et que la troisième languette se met en prise avec un premier bord de l'implant, les première et deuxième languettes des premier et deuxième bras sont configurées pour se mettre en prise avec les deuxième et troisième bords de l'implant adjacents au premier bord de l'implant.

10. Système de pose d'implant selon la revendication 8, dans lequel lorsque l'implant est circulaire et a un bord externe, et la troisième languette se met en prise avec un premier emplacement sur le bord externe, les première et deuxième languettes des premier et deuxième bras sont configurées pour se mettre en prise avec les deuxième et troisième emplacements sur le bord externe de l'implant espacé par rapport au premier emplacement.

11. Ensemble d'implant, comprenant :
un implant plan d'un seul tenant (12) ; et
un système de pose incluant :
une tige externe (242) incluant une extrémité proximale, une extrémité distale et une lumière s'étendant entre elles ;
une tige interne (244) disposée de façon coulissante à l'intérieur de la lumière de la tige externe ; et
une armature (246) attachée fixement à une extrémité distale de la tige interne et configurée pour être attachée de manière détachable à l'implant, l'armature incluant une partie corps (256) et au moins des premier et deuxième bras (260, 264) s'étendant à partir de la partie corps, chacun des premier et deuxième bras ayant une extrémité libre (262, 266) configurée pour pénétrer dans l'implant lorsqu'elle est insérée à travers l'implant dans une première direction, et pour résister au retrait de l'implant lorsqu'elle est tirée dans une deuxième direction opposée à la première direction ;
dans lequel les premier et deuxième bras (260, 264) sont tissés dans et hors de l'implant (12).

12. Ensemble d'implant selon la revendication 11, dans lequel les extrémités libres des premier et deuxième bras incluent chacun une pointe distale acérée (270, 272) et une région évasée (271, 273) proximale de la pointe distale acérée.

13. Ensemble d'implant selon la revendication 11, dans lequel l'armature, incluant la partie corps et les premier et deuxième bras, est formée à partir d'une seule pièce monolithique.

14. Ensemble d'implant selon la revendication 11, dans lequel l'armature définit un arc concave unique entre la partie corps (256) et les extrémités libres (262, 266) des premier et deuxième bras.

15. Ensemble d'implant selon la revendication 11, dans lequel la partie corps et les premier et deuxième bras définissent une forme en Y.
